(19) Europäisches Patentamt
European Patent Office
Office européen des brevets

(11) Numéro de publication : **0 591 027 A1**

# (12) DEMANDE DE BREVET EUROPEEN

(21) Numéro de dépôt : **93402281.5**

(22) Date de dépôt : **20.09.93**

(51) Int. Cl.[5] : **C07D 401/14**, C07D 401/04, C07D 471/04

(30) Priorité : **28.09.92 FR 9211551**

(43) Date de publication de la demande :
**06.04.94 Bulletin 94/14**

(84) Etats contractants désignés :
**AT BE CH DE DK ES FR GB GR IE IT LI LU MC NL PT SE**

(71) Demandeur : **SYNTHELABO**
**22, Avenue Galilée**
**F-92350 Le Plessis Robinson (FR)**

(72) Inventeur : **Jegham, Samir**
**65 rue du Lieutenant Colonel Prudhon**
**F-95100 Argenteuil (FR)**
Inventeur : **Angel, Itzchak**
**19 rue du Bosquet**
**F-94150 Rungis (FR)**
Inventeur : **Purcell, Thomas**
**47 bis rue de la Millière, Les Mesnuls**
**F-78490 Montfort l'Amaury (FR)**
Inventeur : **Schoemaker, Johannes**
**29 allée des Graviers de la Salmouille**
**F-91190 Gif s/Yvette (FR)**

(74) Mandataire : **Thouret-Lemaitre, Elisabeth et al**
**SYNTHELABO, Service Brevets, B.P. 72**
**F-92352 Le Plessis-Robinson Cédex (FR)**

(54) **Dérivés de pipéridine, leur préparation et leur application en thérapeutique.**

(57) Composés répondant à la formule (I)

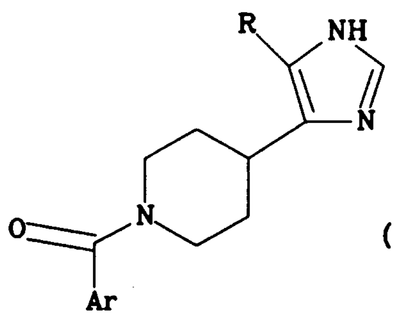

(I)

dans laquelle
R représente soit un atome d'hydrogène, soit un groupe $(C_1-C_6)$alkyle droit ou ramifié et
Ar représente soit un groupe phényle éventuellement substitué par un ou plusieurs radicaux choisis parmi les atomes d'halogènes et les groupes amino, $(C_1-C_2)$alcoxy ou $(C_3-C_6)$ cycloalkyl-$(C_1-C_2)$alcoxy, soit un groupe hétéroaryle,
à l'exception des composés où R est un atome d'hydrogène et Ar un groupe phényle ou un groupe 4-chlorophényle,
ainsi que leurs sels d'addition à des acides pharmaceutiquement acceptables.
Application thérapeutique.

EP 0 591 027 A1

La présente invention a pour objet des dérivés de pipéridine, leur préparation et leur application en thérapeutique.

Les composés de l'invention répondent à la formule (I)

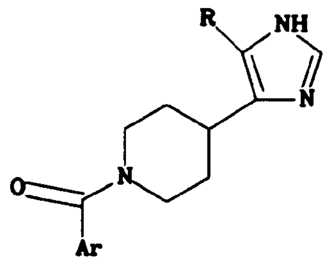

(I)

dans laquelle
R représente soit un atome d'hydrogène, soit un groupe $(C_1-C_6)$alkyle droit ou ramifié et
Ar représente soit un groupe phényle éventuellement substitué par un ou plusieurs radicaux choisis parmi les atomes d'halogènes et les groupes amino, $(C_1-C_2)$alcoxy et
$(C_3-C_6)$cycloalkyl$(C_1-C_2)$alcoxy, soit un groupe hétéroaryle, à l'exception des composés pour lesquels R est un atome d'hydrogène et Ar est un groupe phényle ou un groupe 4-chlorophényle.

Parmi les composés selon l'invention, les composés préférés sont les composés répondant à la formule (I) dans laquelle R représente soit un atome d'hydrogène, soit un groupe $(C_1-C_6)$alkyle droit ou ramifié et Ar représente soit un groupe phényle éventuellement substitué par un ou plusieurs radicaux choisis parmi l'atome de chlore et les groupes amino, méthoxy et cyclopropylméthoxy, soit un groupe imidazo[1,2-*a*]pyridin-2-yle, soit un groupe indol-3-yle, soit un groupe indazol-3-yle éventuellement substitué en position 1 par un radical choisi parmi les groupes $(C_1-C_2)$alkyle et aryl$(C_1-C_2)$alkyle et en position 5 par un radical choisi parmi les atomes d'hydrogène, d'halogènes et le groupe $(C_1-C_2)$alkyle.

Parmi ceux-ci peuvent être cités les composés répondant à la formule (I) dans laquelle
R représente soit un atome d'hydrogène, soit un groupe $(C_1-C_6)$alkyle droit ou ramifié et
Ar représente un groupe indazol-3-yle éventuellement substitué en position 1 par un radical choisi parmi les groupes $(C_1-C_2)$alkyle et aryl$(C_1-C_2)$alkyle et en position 5 par un radical choisi parmi les atomes d'hydrogène, d'halogènes et le groupe $(C_1-C_2)$alkyle.

Les composés selon l'invention peuvent se présenter à l'état de bases libres ou de sels d'addition à des acides pharmaceutiquement acceptables. Les composés dont la formule est une forme mésomère de la formule (I), font partie de l'invention.

La demande de brevet européen 0494010 décrit des composés de formule (I) dans laquelle R est un atome d'hydrogène et Ar est un groupe phényle éventuellement substitué en para par un atome de chlore.

Conformément à l'invention, on peut préparer les composés de formule (I) selon le procédé illustré dans le schéma 1 ci-dessous :

Schéma 1

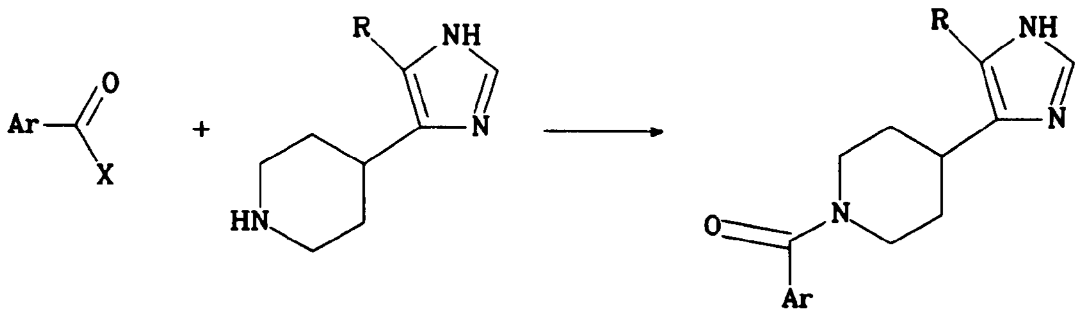

(II) (III) (I)

On fait réagir un composé de formule (II) dans laquelle Ar est tel que défini ci-dessus et X représente soit

un atome d'halogène, par exemple un atome de chlore, soit un groupe hydroxy, avec un dérivé de pipéridine de formule (III) dans laquelle R est tel que défini précédemment.

Les composés de départ sont disponibles dans le commerce ou décrits dans la littérature ou peuvent être préparés selon des méthodes qui y sont décrites ou qui sont connues de l'homme du métier.

L'acide 1*H*-indazole-3-carboxylique est décrit dans *J. Amer. Chem. Soc.,* 1952, 2009.

L'acide 4-amino-5-chloro-2-(cyclopropylméthoxy)benzoïque est décrit dans les brevets britanniques GB 1507462, GB 1088581 et GB 101978.

La 4-(1*H*-imidazol-4-yl)pipéridine est décrite dans *Arch. Pharmaz.,* (Weinheim. Ger.) 1973, **306**(12), 934-42 et dans la demande de brevet européen 0197840.

La 4-(5-méthyl-1*H*-imidazol-4-yl)pyridine est décrite dans *J. Med. Chem.,* 1986, **29**, 2154-63.

Les exemples qui suivent illustrent en détail la préparation des composés selon l'invention.
Les microanalyses et les spectres IR et RMN confirment la structure des composés obtenus.

## Exemple 1

1-(3,5-dichlorobenzoyl)-4-(1*H*-imidazol-4-yl)pipéridine, fumarate

On dissout 0,469 g (2,5 mmoles) de monochlorhydrate de 4-(1*H*-imidazol-4-yl)pipéridine dans 5 ml de soude 1 N à 0 °C. On ajoute ensuite 0,524 g (2,5 mmoles) de chlorure de 3,5-dichlorobenzoyle et on agite, à 0 °C pendant 15 minutes. On filtre le précipité obtenu, on le lave par de la soude 1 N puis par de l'eau et on le sèche. On recristallise le résidu dans de l'éthanol.
On obtient 0,4 g de produit.
Point de fusion = 240-242 °C
On prépare le fumarate en dissolvant la base dans l'éthanol, puis en ajoutant un équivalent d'acide fumarique.
On recristallise le fumarate dans un mélange d'isopropanol et d'éthanol.
Point de fusion = 178-183 °C

## Exemple 2

4-(1*H*-imidazol-4-yl)-1-((1*H*-indol-3-yl)carbonyl]pipéridine, fumarate

On ajoute 0,81 ml (5,82 mmoles) de triéthylamine à une suspension de 0,48 g (3 mmoles) d'acide 1*H*-indole-3-carboxylique et de 0,453 g (3 mmoles) de 4-(1*H*-imidazol-4-yl)pi-péridine dans 10 ml de dichlorométhane, à la température ambiante et sous argon. On ajoute 1,29 ml (6 mmoles) d'azoture de diphénylphosphoryle et on agite pendant 20 heures. On extrait le milieu réactionnel par de l'acétate d'éthyle en milieu acide. On récupère la phase aqueuse, on l'alcalinise avec une solution de carbonate de potassium et on l'extrait par de l'acétate d'éthyle. On récupère la phase organique, on la lave à l'eau puis avec une solution saturée en chlorure de sodium. On la sèche sur du sulfate de magnésium. On purifie le résidu obtenu par chromatographie sur colonne de gel de silice en éluant par un mélange dichlorométhane/méthanol (90/10). On évapore les fractions pures et on recueille 0,27 g de produit.
Pour préparer le fumarate, on reprend la base par de l'éthanol et on ajoute un équivalent d'acide fumarique.
Après recristallisation dans un mélange d'éthanol et d'éther isopropylique, on filtre et on sèche le produit obtenu sous forme d'hémifumarate.
On obtient 0,3 g de produit.
Point de fusion = 220 °C (déc)  Rendement = 28 %

## Exemple 3

1-((1*H*-indazol-3-yl)carbonyl)-4-(5-méthyl-1*H*-imidazol-4 yl)pipéridine, fumarate

Dans un ballon de 100 ml, on place 1,35 g (8,15 mmoles) de 4-(5-méthyl-1*H*-imidazol-4-yl)pipéridine dans 15 ml de dichlorométhane et 4 ml de diméthylformamide. On ajoute 1,32 g (8,15 mmoles) d'acide 1*H*-indazole-3-carboxylique et 2,2 ml de triéthylamine. On laisse sous agitation pendant 5 minutes. On ajoute 3,5 ml d'azoture de diphénylphosphoryle et on laisse sous agitation pendant 72 heures. On ajoute de l'acétate d'éthyle et on extrait 3 fois par de l'acide chlorhydrique 2 N. On récupère la phase aqueuse que l'on alcalinise avec une solution de carbonate de sodium. On extrait 3 fois par de l'acétate d'éthyle, on recueille la phase organique, on la sèche et on l'évapore à sec. On purifie le résidu par chromatographie sur colonne de gel de silice en éluant par un mélange de dichlorométhane/méthanol/ammoniaque (90/10/1).
On récupère 1 g de produit sous forme de base pure.
On prépare le fumarate comme décrit dans l'exemple 1.
Point de fusion = 213-215 °C  Rendement = 32 %

Le tableau suivant illustre les structures chimiques et les propriétés physiques de quelques composés selon l'invention.

*Légende du tableau*

dans la colonne "F (°C)" du tableau

(déc) signifie décomposition

dans la colonne "Sel" du tableau

(x:y) signifie x moles d'acide pour y moles de base, l'absence de toute mention signifie que le composé est à l'état de base,

chlor. représente le chlorhydrate

fum. représente le fumarate.

méthanesulf. représente le méthanesulfonate

## Tableau

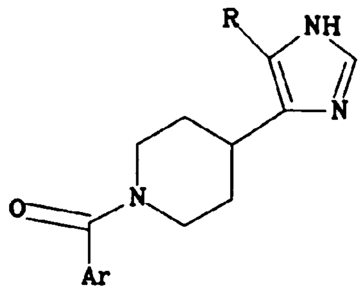

(I)

| N° | R | Ar | F (°C) | Sel |
|---|---|---|---|---|
| 1 | -H | 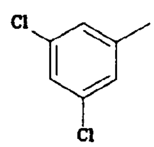 | 178-183 | fum. (1:2) |
| 2 | -H | 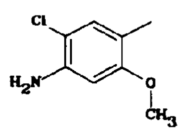 | 140-145 | - |
| 3 | $-CH_3$ | 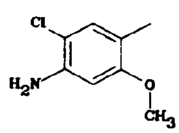 | 135-145 | - |
| 4 | -H | 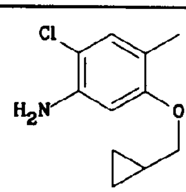 | 135 (déc) | - |
| 5 | -H | 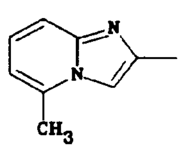 | > 220 (déc) | fum. (1:1) |
| 6 | -H | 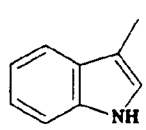 | 220 (déc) | fum. (1:2) |

| N° | R | Ar | F (°C) | Sel |
|---|---|---|---|---|
| 7 | $-CH_3$ | 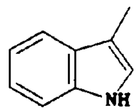 | 175-180 | fum. (1:1) |
| 8 | -H | 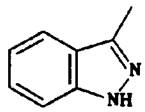 | 210 (déc) | fum. (1:2) |
| 9 | $-CH_3$ | 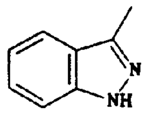 | 202 | - |
| 10 | $-CH_3$ | 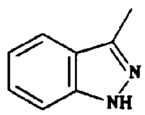 | 213-215 | fum. (1:2) |
| 11 | $-CH_3$ | 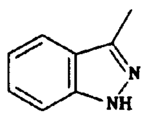 | 235-237 | méthanesulf. (1:1) |
| 12 | $-(CH_2)_2CH_3$ | 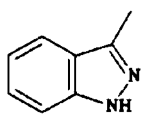 | 217-222 | fum. (1:1) |
| 13 | $-CH(CH_3)_2$ | 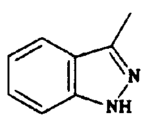 | 239-241 | fum. (1:1) |
| 14 | $-(CH_2)_3CH_3$ | 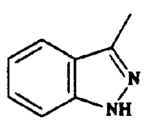 | 220-224 | fum. (1:1) |
| 15 | -H | 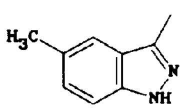 | 185-192 | fum. (1:1) |

| N° | R | Ar | F (°C) | Sel |
|---|---|---|---|---|
| 16 | $-CH_3$ | 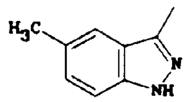 | 186-192 | fum. (1:1) |
| 17 | -H | 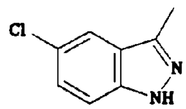 | 188-195 | fum. (1:1) |
| 18 | $-CH_3$ | 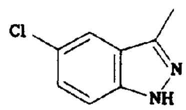 | 206-212 | fum. (1:1) |
| 19 | $-(CH_2)_3CH_3$ | 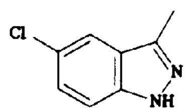 | 130-135 | chlor. (1:1) |
| 20 | $-CH_3$ | 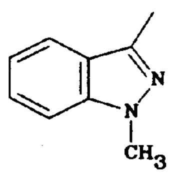 | 181-182 | - |
| 21 | $-CH_2CH_3$ | 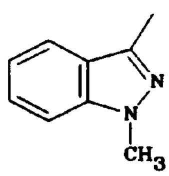 | 182-184 | - |
| 22 | -H | 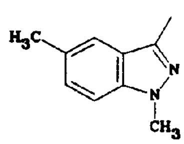 | 172-175 | fum. (1:1) |

| N° | R | Ar | F (°C) | Sel |
|---|---|---|---|---|
| 23 | $-(CH_2)_3CH_3$ | 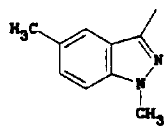 | 217-220 | - |
| 24 | -H | 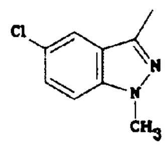 | 186-192 | fum. (1:1) |
| 25 | $-CH_3$ | 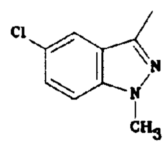 | 218-225 | fum. (1:1) |
| 26 | $-(CH_2)_3CH_3$ | 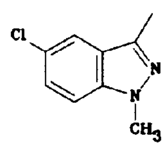 | > 250 | - |
| 27 | -H | 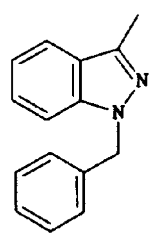 | 165-167 | fum. (1:1) |

Les composés de l'invention ont fait l'objet d'essais pharmacologiques qui ont montré leur intérêt comme substances actives en thérapeutique.

Ainsi ils ont été testés quant à leurs effets sur l'accumulation d'$AMP_c$ dans une préparation de culture primaire de neurones de colliculi d'embryons de souris selon la technique décrite par Dumuis et coll. *Mol. Pharmacol.*, **34**, 880-887, 1988. Cette accumulation est représentative de l'activité de l'adénylcyclase à laquelle sont positivement couplés les récepteurs sérotoninergiques de type 5-$HT_4$.

On prélève les colliculi sur des embryons de souris âgés de 14 à 15 jours. On dissocie les neurones mécaniquement et on les met en culture, dans des boîtes Costar™ de 12 puits à raison de $10^6$ cellules par puits, dans un milieu nutritif DMEM/F12™ avec des suppléments mais sans sérum. On maintient les cultures à 37 °C sous une atmosphère humidifiée (5 % $CO_2$/ 95 % air).

Six jours après le début de la culture, on incube les cellules pendant 2 heures, dans le milieu de culture précédemment décrit, en présence de 0,1 nmole d'adénine tritiée (activité spécifique 20 Ci/mmole) par puits. On lave les cellules avec le milieu de culture et on réalise une deuxième incubation, dans le milieu de culture, en présence d'isobutylméthylxanthine (0,75 mM), de forskoline (0,1 μM) et des produits à tester à différentes concentrations, dans un volume final de 1 ml par puits. Après 10 minutes d'incubation, on arrête la réaction par aspiration du milieu et addition de 1 ml d'acide trichloracétique à 5 %. On décolle les neurones, on les homogénéise aux ultrasons et on les centrifuge à 8000 g pendant 2,5 minutes. On recueille le surnageant et on ajoute 100 μl d'une solution contenant de l'$AMP_c$ (5 mM) et de l'ATP (5 mM). On sépare l'ATP et l'$AMP_c$ tritiés formés, par passage sur résine DOWEX™ AG50WX8 puis sur alumine.

Les résultats sont exprimés en % $AMP_c[H^3]/ATP[H^3]$.

Les $CE_{50}$ et les $CI_{50}$ représentent respectivement les concentrations qui produisent la moitié de la stimulation et de l'inhibition maximales.

Les composés de l'invention les plus actifs dans cet essai se caractérisent par des $CI_{50}$ comprises entre 1 et 10 μM.

Les composés de l'invention ont été également testés *in vivo* quant à leur effet sur la diarrhée induite par le 5-HTP chez la souris selon la technique décrite par Warrick et coll. *J. Pharm. Pharmacol.*, **33,** 675-676, 1981. On utilise des souris $CD_1$ mâles, pesant 25-30 g et à jeun depuis 18 heures. On administre les composés ou le véhicule 20 minutes (voie intrapéritonéale) ou 60 minutes (voie orale) avant l'injection par voie intrapéritonéale de 5-HTP à la dose de 25 mg/kg. On place les animaux dans des cages individuelles et on les observe pendant 3 heures en notant le nombre d'animaux ayant de la diarrhée 30 minutes, 1 heure, 2 heures et 3 heures après l'administration de 5-HTP.

Les résultats sont exprimés en pourcentage d'animaux protégés par le prétraitement comparativement aux animaux témoins ayant reçu le véhicule en prétraitement.

Les composés de l'invention les plus actifs dans cet essai inhibent la diarrhée induite par le 5-HTP après une dose administrée de 0,002 mg/kg par voie intrapéritonéale ou de 0,1 mg/kg par voie orale.

Les composés selon l'invention ont également été testés quant à leurs effets inhibiteurs de la liaison de la $[^3H]$quipazine avec les récepteurs sérotoninergiques de type $5\text{-}HT_3$ présents dans le cortex cérébral du rat, selon une variante de la méthode décrite par Milburn et Peroutka (*J. Neurochem.*, **52**, 1787-1792, 1989).

On utilise des rats mâles Sprague-Dawley, de 150 à 200 g, dans tous les essais. On en prélève le cortex cérébral et on l'homogénéise dans 20 volumes (poids/volume) de tampon Hepes 25 mM ou de tampon Hepes 25 mM contenant du chlorure de sodium (180 mM), du chlorure de calcium (2,5 mM), du chlorure de potassium (5 mM) et du chlorure de magnésium (1,2 mM) (pH=7,4), à l'aide d'un broyeur Polytron™. Après centrifugation de la suspension pendant 10 minutes à 45000xg, on remet le culot en suspension dans le volume initial de tampon, contenant éventuellement 0,05% de Triton X-100™, et on effectue une première incubation de 30 minutes à 37°C. On effectue ensuite encore deux centrifugations comme précédemment décrit, et on reprend le culot final dans 11,7 volumes de tampon Hepes 25 mM à pH=7,4.

On détermine la liaison de la $[^3H]$quipazine (51,6-69,8 Ci/mmole, New England Nuclear, Boston, Ma, USA) en faisant incuber 150 μl de la suspension membranaire avec le radioligand (0,8 nM) dans un volume final de 1 ml, pendant 30 minutes à 25°C, en absence ou en présence du composé à étudier. L'incubation a lieu en présence de 0,1 μM de paroxétine et de 1 μM de kétansérine. La liaison non spécifique est déterminée en présence de 1 μM d'ondansetron. Après l'incubation, on dilue le mélange testé avec 5 ml de tampon Tris-HCl glacé 50 mM (pH=7,4 à 0°C). On collecte les membranes par filtration sur des filtres Whatman GF/B™ prétraités avec 0,05% de polyéthylèneimine, et on les lave avec trois volumes de 5 ml de tampon Tris-HCl glacé 50 mM.

La radioactivité retenue sur les filtres est mesurée par spectrométrie de scintillation liquide à une efficacité de 50 à 60%.

Les résultats s'expriment par la concentration ($CI_{50}$) de composé étudié qui inhibe 50% de la liaison de la $[^3H]$quipazine, déterminée par une méthode graphique ou mathématique.

Les composés de l'invention les plus actifs dans cet essai se caractérisent par des $CI_{50}$ inférieures à 1 nM ($10^{-9}$M).

Les résultats des essais biologiques montrent que les composés de l'invention sont des ligands des récepteurs séro-toninergiques de types $5\text{-}HT_3$ et $5\text{-}HT_4$.

Ils peuvent donc être utilisés pour le traitement et la prévention des désordres dans lesquels les récepteurs $5\text{-}HT_3$ et $5\text{-}HT_4$ sont impliqués, tels que nausées et vomissements, par exemple consécutifs à un traitement antitumoral ou à l'administration d'un anesthésique ; troubles du système nerveux central tels que la schizophrénie, la manie, l'anxiété et la dépression ; troubles de la cognition tels que la démence sénile ou présénile d'Alzheimer ; dyskinésie, douleurs, migraines et maux de tête ; troubles de la dépendance ou du sevrage d'alcool ou de drogues ; troubles de la fonction gastrointestinale tels que dyspepsie, ulcère peptique, aigreurs d'estomac, flatulences ; troubles du système cardiovasculaire et troubles respiratoires.

Ils peuvent également être utilisés pour le traitement et la prévention des désordres tels que la diarrhée, le colon irritable, le reflux oesophagien, les troubles moteurs intestinaux, les troubles de la sécrètion intestinale, la fibrose kystique du pancréas, le syndrome carcinoïde et l'incontinence.

A cet effet ils peuvent être présentés sous toutes formes appropriées à l'administration orale ou parentérale, telles que comprimés, dragées, gélules, capsules, suspensions ou solutions buvables ou injectables, etc. en association avec des excipients convenables, et dosées pour permettre une administration de 0,005 à 10 mg 1 à 4 fois par jour.

## Revendications

1. Composés répondant à la formule (I)

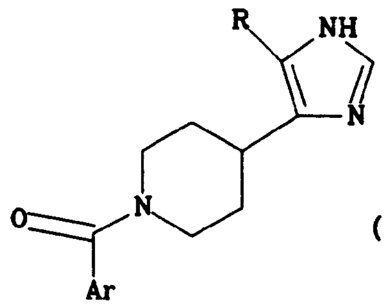

(I)

dans laquelle
R représente soit un atome d'hydrogène, soit un groupe $(C_1-C_6)$alkyle droit ou ramifié et
Ar représente soit un groupe phényle éventuellement substitué par un ou plusieurs radicaux choisis parmi les atomes d'halogènes et les groupes amino, $(C_1-C_2)$alcoxy ou $(C_3-C_6)$cycloalkyl$(C_1-C_2)$alcoxy, soit un groupe hétéroaryle,
à l'exception des composés où R est un atome d'hydrogène et
Ar un groupe phényle ou un groupe 4-chlorophényle, ainsi que leurs sels d'addition à des acides pharmaceutiquement acceptables.

2. Composés selon la revendication 1 caractérisés en ce que R représente soit un atome d'hydrogène, soit un groupe $(C_1-C_6)$alkyle droit ou ramifié et
Ar représente soit un groupe phényle éventuellement substitué par un ou plusieurs radicaux choisis parmi l'atome de chlore et les groupes amino, méthoxy et cyclopropylméthoxy, soit un groupe imidazo[1,2-*a*]pyridin-2-yle, soit un groupe indol-3-yle, soit un groupe indazol-3-yle éventuellement substitué en position 1 par un radical choisi parmi les groupes $(C_1-C_2)$alkyle et aryl$(C_1-C_2)$alkyle et en position 5 par un radical choisi parmi les atomes d'hydrogène, d'halogènes et le groupe $(C_1-C_2)$alkyle.

3. Composés selon la revendication 2 caractérisés en ce que R représente soit un atome d'hydrogène, soit un groupe $(C_1-C_6)$alkyle droit ou ramifié et
Ar représente un groupe indazol-3-yle éventuellement substitué en position 1 par un radical choisi parmi les groupes $(C_1-C_2)$alkyle et aryl$(C_1-C_2)$alkyle et en position 5 par un radical choisi parmi les atomes d'hydrogène, d'halogènes et le groupe $(C_1-C_2)$alkyle.

4. Procédé de préparation des composés selon la revendication 1, procédé caractérisé en ce que l'on fait réagir un composé de formule (II)

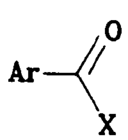

(II)

dans laquelle Ar est tel que défini dans la revendication 1 et X représente soit un atome d'halogène, soit un groupe hydroxy, avec un dérivé de pipéridine de formule (III)

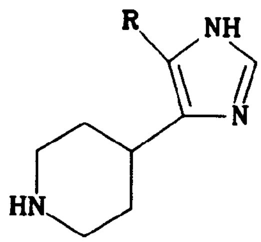

(III)

dans laquelle R est tel que défini dans la revendication 1.

**5.** Médicament caractérisé en ce qu'il contient un composé selon l'une quelconque des revendications 1 à 3.

**6.** Composition pharmaceutique, caractérisée en ce qu'elle contient un composé selon l'une quelconque des revendications 1 à 3, en association avec tout excipient approprié.

Office européen des brevets

# RAPPORT DE RECHERCHE EUROPEENNE

Numero de la demande

EP 93 40 2281

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X,D | EP-A-0 494 010 (INSERM)<br>* exemples 15,16 * | 1,5,6 | C07D401/14<br>C07D401/04<br>C07D471/04 |
| A | EP-A-0 197 840 (INSERM)<br>* exemples 2,32,47,66 * | 1-6 | |
| A,D | J. MED. CHEM.<br>vol. 29, no. 11, Novembre 1986,<br>pages 2154 - 2163<br>C.A. LIPINSKI ET AL.<br>* exemples 4,8 * | 1-6 | |
| A,D | ARCHIV DER PHARMAZIE<br>vol. 306, no. 12, Décembre 1973, WEINHEIM DE<br>pages 934 - 942<br>W. SCHUNACK<br>* exemple 4c * | 1-6 | |
| | | | **DOMAINES TECHNIQUES RECHERCHES (Int. Cl.5 )** |
| | | | C07D<br>A61K |

Le présent rapport a été établi pour toutes les revendications

| Lieu de la recherche | Date d'achèvement de la recherche | Examinateur |
|---|---|---|
| BERLIN | 10 NOVEMBRE 1993 | FRELON D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)